# EUROPEAN PATENT APPLICATION

(11) **EP 2 022 523 A1**
(43) Date of publication of application: **11.02.2009**
(21) Application number: 08161520.5
(22) Date of filing: 31.07.2008
(51) Int. Cl.: A61M 5/32, A61M 5/31, A61M 5/28

(54) **Combined container-syringe with needle protection**

(30) Priority: 02.08.2007 JP 2007201959
(71) Applicant: ARTE CORPORATION, Tokyo (JP)
(72) Inventor: Kakiuchi, Makoto Arte Corporation, Takahagi-shi Ibaraki (JP)
(74) Representative: Metman, Karel Johannes

(57) **Abstract**

The present invention relates to a combined container-syringe including: a cylindrical cylinder (7) filled with a drug solution; a cylindrical tip (8) provided at the cylinder; an injection needle (10) mounted on the cylindrical tip; a finger grip (9) provided on the cylinder; a cylindrical-shaped safety device (4) that is mounted in a slidable manner on the outer periphery of the cylinder and covers the injection needle when it has moved in the front end direction; a retaining member (17,18) that selectively engages the safety device at a first position that is away from the injection needle and a second position that covers the injection needle; and a coil spring that biases the safety device in the direction of the second position. According to the present invention, it is possible to reliably cover the injection needle by causing the safety device to move with respect to the syringe body and so possible to ensure safety.

## Description

### BACKGROUND OF THE INVENTION

### Field of the Invention

The present invention relates to a combined container-syringe that is provided with a safety device that is used for covering the injection needle after the completion of injection and the like and ensuring safety.

### Description of Related Art

Since a combined container-syringe can be used immediately after being taken out from the packaging without performing troublesome procedures at medical institutions due to the fact that the drug solution has been prefilled, it is very convenient and very useful for lightening the workload of people involved in medical service such as physicians and nurses. For this reason, it is being adopted by many medical facilities.

In the past, when people involved in medical service used syringes, they sometimes accidentally stuck themselves with the injection needle after being used for injection, and as a result of doing so faced the risk of a viral infection. For that reason, with the aim of safely processing injection needles after injection into patients, a syringe has been proposed that is equipped with a safety device as a cylindrical cover that prevents careless contact with the injection needle by covering the injection needle therewith after injection. For example, according to the syringe that is disclosed in Japanese Unexamined Patent Application, Publication No. 2001-29334, a safety device having flexibility is engaged on an outer circumference of a cylinder, and by applying pressure to the safety device to be flexed, the engagement state is released. By pointing the injection needle upward while holding the syringe by the safety device, the syringe body moves downward by its own weight, whereby the injection needle comes to be covered with the safety device.

However, in the syringe that is equipped with a conventional safety device as described above, the means that causes the safety device to move to a position that covers the injection needle is merely the syringe body's own weight. Due to this fact, the force of relative movement of the safety device with respect to the needle is weak, and depending on the circumstances the safety device may become caught on the syringe body in the middle of covering the injection needle. As a result, the syringe that is equipped with the conventional safety device has the problems that the safety device is not able to reliably cover the injection needle, whereby not ensuring the safety.

The present invention was achieved in view of the above circumstances, and has as its object to provide a combined container-syringe that can ensure safety by reliably causing the safety device to move with respect to the syringe body to a state of covering the injection needle.

### SUMMARY OF THE INVENTION

In order to solve the abovementioned problem, this invention proposes the following means.

The combined container-syringe according to the present invention has a cylindrical cylinder that is filled with a drug solution; a cylindrical tip provided at the front end side of the cylinder; an injection needle mounted on the front end of the cylindrical tip; a finger grip that is provided on the rear end side of the cylinder; a cylindrical-shaped safety device that is mounted in a slidable manner on the outer periphery of the cylinder, and covers the injection needle when it has moved in the front end direction; a retaining member that selectively engages the safety device at a first position that is away from the injection needle and a second position that covers the injection needle; and a biasing member that biases the safety device in the direction of the second position.

When the engagement of the safety device at the first position by the retaining member is released, the safety device moves in the direction of the second position by the biasing force of the biasing member such that the safety device is engaged by the retaining member at the second position. Thereby, it is possible to reliably cause the safety device to move to a position of covering the injection needle, and so it is possible to reliably cover the injection needle with the safety device.

Also, in the combined container-syringe according to the present invention, it is preferable that the biasing member is a coil spring; and in the state of the safety device being fixed to the outer periphery of the cylinder, the coil spring is mounted in a compressed state between the cylindrical tip and a spring receiving member that is provided at the front end of the safety device.

When the engagement of the safety device at the first position by the retaining member is released, the coil spring that is provided in a compressed state between the cylindrical tip and the spring receiving member at the front end of the safety device expands in the direction of the front end of the combined container-syringe body with respect to the cylindrical tip, and accompanying this the safety device moves in the direction of the front end of the combined container-syringe body. Thereby, it is possible to reliably cause the safety device to move to the second position of covering the injection needle.

Also, in the combined container-syringe according to the present invention, it is preferable that the biasing member is a coil spring; and in the state of the safety device being fixed to the outer periphery of the cylinder, the coil spring is mounted in a compressed state between the finger grip and a spring receiving portion that is formed on an inner circumferential wall surface on the rear end side of the safety device.

When the engagement of the safety device at the first position by the retaining member is released, the coil spring that is provided in a compressed state between the spring receiving portion that is formed on the inner circumferential wall surface on the rear end side of the safety device and the finger grip that is fixed to the rear end side of the combined container-syringe body expands in the direction of the front end of the combined container-syringe body with respect to the finger grip, and accompanying this the safety device moves in the direction of the front end of the combined container-syringe body. Thereby, it is possible to reliably cause the safety device to move to the second position of covering the injection needle.

Moreover, in the combined container-syringe according to the present invention, it is preferable that the retaining member comprises a first retaining member that engages the safety device in a disengagable manner at the first position; and a second retaining member that engages the safety device at the second position.

Since the first retaining member is disengagable, it is possible to readily release the engagement of the safety device at the first position. And when the safety device has moved to the second position, the safety device is reliably engaged by the second retaining member. Accordingly, it is possible to ensure safety with no inadvertent movement of the safety device.

According to the combined container-syringe of to the present invention, since it is possible to reliably cause the safety device to move with respect to the combined container-syringe body, it is possible to reliably cover the injection needle with the safety device, and so possible to ensure safety.

### BRIEF DESCRIPTION OF THE DRAWINGS

FIG. 1 is a side view of the combined container-syringe according to one embodiment of the present invention.
FIG. 2 is a vertical sectional view of the combined container-syringe body.
FIG. 3 is a vertical sectional view of the safety device body.
FIG. 4 is a view along arrow A in FIG. 3 in the vicinity of the return prevention stoppers.
FIG. 5 is a vertical sectional view of the press ring.
FIG. 6 is a vertical sectional view of the spring receiving member.
FIG. 7 is a vertical sectional view of the safety device.
FIG. 8 is a vertical sectional view of the state of the safety device being mounted on the combined container-syringe body.
FIG. 9 is a vertical sectional view of the state of the safety device being mounted on the combined container-syringe body and the pressing ring having been moved.
FIGS. 10A and 10B are explanatory drawings of when mounting the coil spring and the spring receiving member on the combined container-syringe body and the safety device.
FIG 11 is an enlarged view of the vicinity of the engagement portion, the return prevention stopper, and the slip-out prevention stopper in FIG. 1.
FIG. 12 is a side view of the combined container-syringe according to a modification example.
FIG. 13 is a side view of the combined container-syringe according to a modification example.

### DETAILED DESCRIPTION OF THE INVENTION

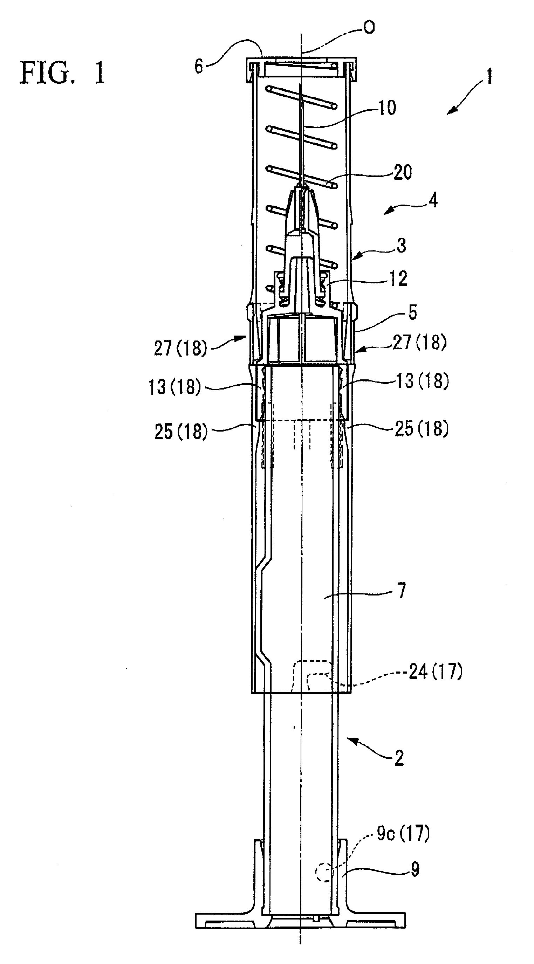

An embodiment of the combined container-syringe according to the invention shall be described hereinbelow with reference to FIG. 1 to FIG. 11. As shown in FIG. 1, a combined container-syringe 1 in the present embodiment is constituted by a combined container-syringe body 2; a safety device 3 that consists of a safety device body 4 and a pressing ring 5; a spring receiving member 6 that is provided at the front end of the safety device 3; and a coil spring 20. Also, hereinbelow, in FIG. 1 the upper side along an axis O of the combined container-syringe body 2 at which an injection needle 10 is provided shall be called the front end, and the lower side at which a finger grip 9 is provided shall be called the rear end.

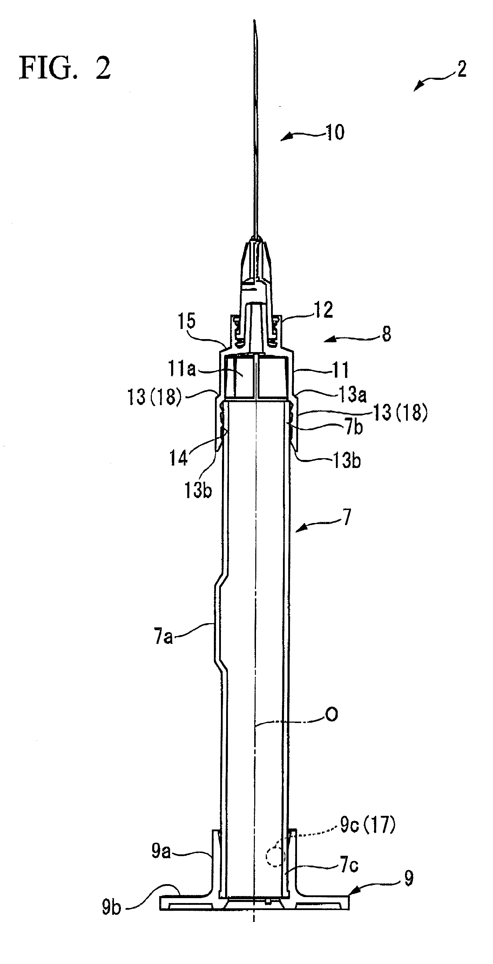

As shown in FIG. 2, the combined container-syringe body 2 is constituted by a cylinder 7, a cylindrical tip 8 that is fitted on the outer periphery of the front end side of the cylinder 7, the finger grip 9 made of a synthetic resin that is fitted on the outer periphery of the rear end side of the cylinder 7, and the injection needle 10 that is mounted on a luer-lock portion 12 that is provided at the front end of the cylindrical tip 8.

The cylinder 7 is made for example of transparent glass, and has a cylindrical shape that extends along the axis O. In the cylinder 7, a bypass path 7a is formed that bulges outward along a predetermined length in the axis direction, and a stopper (not illustrated) is provided at a total of three locations, namely, the front end side and rear end side of the cylinder 7 and directly on the rear end side of the bypass path 7a. A drug solution is sealed inside of the cylinder 7. Also, the front end portion of a plunger rod (not illustrated) that is inserted from the rear end side of the cylinder 7 is connected by being screwed into the stopper on the rear end side.

The cylindrical tip 8 is for example made with a transparent synthetic resin. As shown in FIG. 2, the cylindrical tip 8 is provided with a cylinder portion 11, the cylindrical luer-lock portion 12 that is integrally formed at the front end of the cylinder portion 11 and is an attachment portion of the injection needle 10, and a cylindrical engagement portion 13 that is integrally formed at the rear end of the cylinder portion 11 and has a larger diameter than it. A bypass chamber 11a is provided at the cylinder portion 11. The bypass chamber 11a has an inner diameter that is equal to or slightly larger than the inner diameter of the cylinder 7. The stopper (not illustrated) is inserted into the bypass chamber 11a. The inner hole of the luer-lock portion 12 communicates with the bypass chamber 11a. By fitting an inner hole 14 of the engagement portion 13 on the outer periphery of the front end portion 7b of the cylinder 7, the cylindrical tip 8 is fitted on the cylinder 7.

Also, the front end side of the engagement portion 13 at the cylindrical tip 8 is fashioned into an engagement step portion 13a at which a step portion is formed, and the rear end side is made into an engagement end portion 13b. Note that this engagement portion 13 constitutes a portion of a second retaining member 18 described below. A step portion that faces the front end side, extending from the luer-lock portion 12 to the cylinder portion 11 having a larger diameter than the luer-lock portion 12, serves as a spring abutting portion 15 against which one end of the coil spring 20 described below is abutted.

The finger grip 9 has a fitting portion 9a having a cylindrical shape and a flange 9b that is integrally formed on the rear end portion of the fitting portion 9a and extends to the outer side in the radial direction. The fitting portion 9a is fitted on the outer periphery of the rear end portion 7c of the cylinder 7. Also, an engagement projection 9c is formed on the outer periphery of the fitting portion 9a of the finger grip 9 so as to project to the outer side in the radial direction.

Since the drug solution is filled in advance in the combined container-syringe body 2 having the above constitution, at the time of use it can be used immediately after being taken out from the packaging. Also, as shown in FIG. 1, the safety device 3 having the safety device body 4, the pressing ring 5, and the spring receiving member 6 is mounted on the outer periphery of the combined container-syringe body 2.

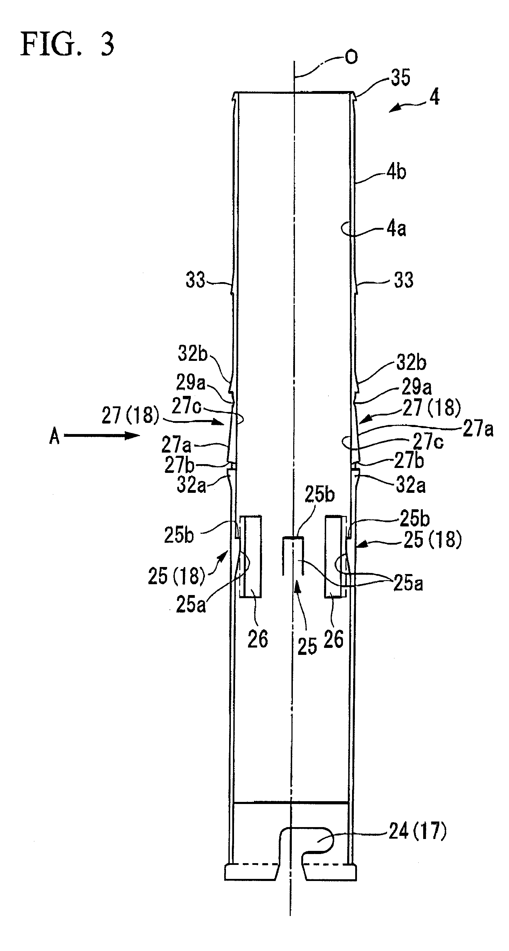

The safety device body 4 is made with a material that is transparent and has moderate flexibility such as polypropylene and thinly fabricated in a range that can maintain strength. As shown in FIG. 3, the safety device body 4 has an approximately cylindrical shape with the same axis O as the combined container-syringe body 2 serving as the longitudinal direction.

As shown in FIG. 3, an L-shaped slit 24 is formed at the rear end of the safety device body 4 by cutting out an approximate L shape in the safety device body 4. The width of this L-shaped slit 24 is approximately the same as the width of the above-mentioned engagement projection 9c. The L-shaped slit 24 and the engagement projection 9c together constitute a first retaining member 17 that fixes the safety device 3 to the outer periphery of the cylinder 7, that is, fixes the safety device 3 to a first position that is separated from the injection needle 10. Also, a slip-out prevention stopper 25 is provided at a total of four locations on the safety device body 4 uniformly spaced in the circumferential direction, and further to the front end side than the L-shaped slit 24. Each slip-out prevention stopper 25 has a sloping surface 25a that is formed so as to gradually reduce the diameter of an inner circumferential wall 4a toward the front end and an engagement surface 25b that is perpendicular to the inner circumferential wall 4a, extending from the apex of the sloping surface 25a to the inner circumferential wall 4a. Moreover, a total of four slits 26 are provided at intermediate positions between every two adjacent slip-out prevention stoppers 25 along the circumferential direction of the inner circumferential wall 4a, with each slit 26 extending along the axis O and opening so as to make the inner circumferential wall 4a communicate with the outer circumferential wall 4b of the safety device body 4.

Moreover, a return prevention stopper 27 is provided at two locations on the outer circumferential wall 4b of the safety device body 4 further to the front end side than the slip-out prevention stopper 25 and the slits 26. Each return prevention stopper 27 has an outer circumferential surface 27a that slopes so as to expand the diameter of the outer circumferential wall 4b toward the rear end direction, an engagement surface 27b that extends from the outer circumferential surface 27a to the inner side in the radial direction, and an inner circumferential surface 27c that is positioned on the reverse side of the outer circumferential surface 27a so as to be flush with the inner circumferential wall 4a of the safety device body 4.

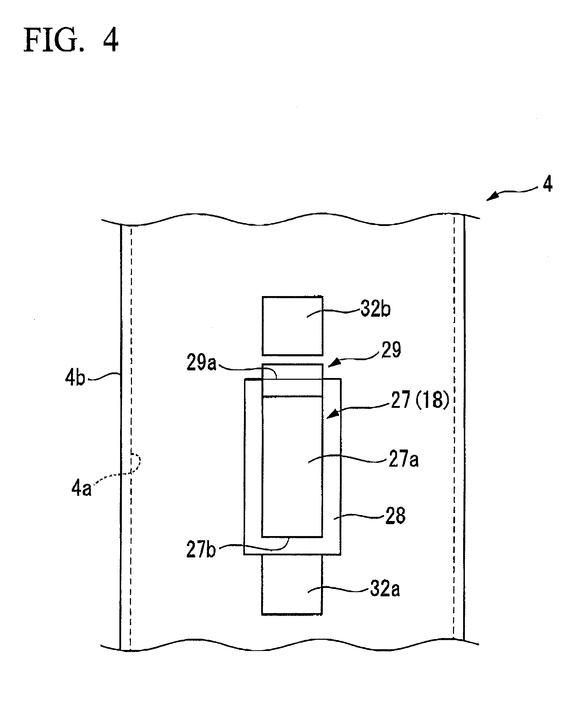

As shown in FIG. 4, each return prevention stopper 27 is surrounded on the rear end side and both sides of the outer circumferential surface 27a in the circumferential direction by a U-shaped slit 28 that is cut out so as to make the inner circumferential wall 4a communicate with the outer circumferential wall 4b. By this U-shaped slit 28, the return prevention stopper 27 is connected with the outer circumferential wall 4b of the safety device body 4 only at the front end side of the outer circumferential surface 27a, with a recessed groove 29a formed at this connection location 29. Thereby, when a force is applied to the return prevention stopper 27 from the outer side in the radial direction, the return prevention stopper 27 is flexed in the manner of a flat spring toward the inner side of the safety device body 4, with the recessed groove 29a of the connection location 29 serving as a fulcrum point.

Note that the second retaining member 18 that fixes the safety device 3 at a location that covers the injection needle 10 is constituted by the slip-out prevention stopper 25, the return prevention stopper 27, and the abovementioned engagement portion 13.

Ring secondary stop projections 32a, 32b are provided at the rear end side of the U-shaped slit 28 and the front end side of the connection location 29, respectively, at each return prevention stopper 27 so as to sandwich the return prevention stopper 27 from the front end side and the rear end side. The ring secondary stop projection 32b that is positioned at the front end side has a slope that is formed so as to increase the diameter of the outer circumferential wall 4b toward the rear end direction. Also, as shown in FIG. 3, two ring primary stop projections 33 that project to the outer side in the radial direction are provided at opposing positions in the circumferential direction on the outer circumferential wall 4b of the safety device body 4, being positioned further to the front end side than the front end side ring secondary stop projection 32b. Also, an engagement edge portion 35 that is formed so that the outer circumferential wall 4b expands in diameter is formed at the front end portion of the safety device body 4.

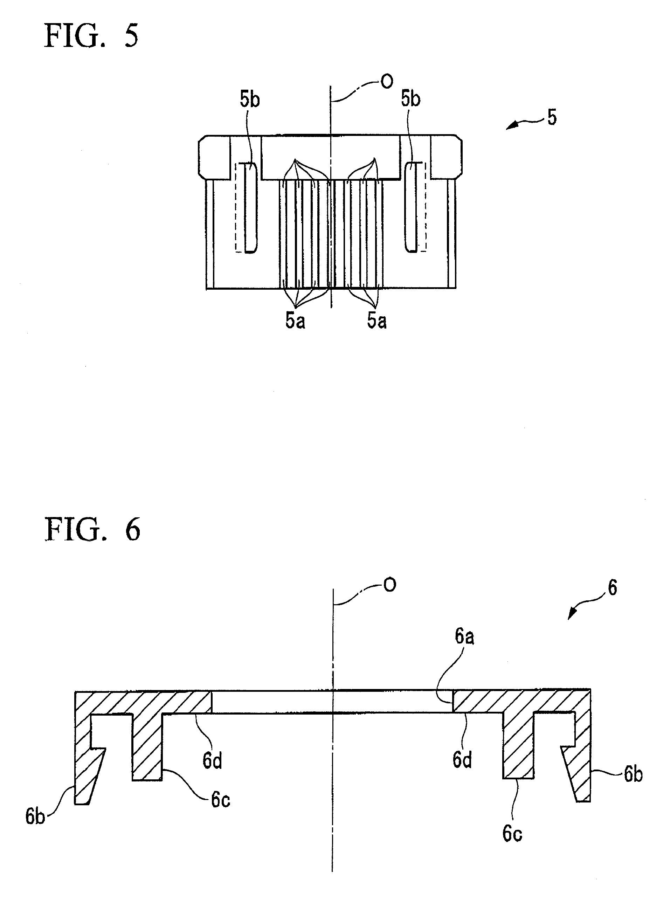

The pressing ring 5, as shown in FIG 5, has a cylindrical shape in which the length in the axis O direction is sufficiently shorter than the safety device body 4, and similarly to the safety device body 4, the pressing ring 5 is made with a material that is transparent and has moderate flexibility such as polypropylene and thinly fabricated in a range that can maintain strength. Also, vertical ribs 5a for preventing slippage are suitably formed on the outer circumference, extending along the axis O and projecting to the outer side in the radial direction. Note that the internal diameter of the pressing ring 5 is approximately the same as or slightly larger than the outer diameter of the safety device body 4. Also, in order to increase flexibility in the pressing ring 5, slits 5b are provided along the axis O.

The spring receiving member 6, as shown in FIG. 6, has a disk shape that is provided with an opening portion 6a in the center for allowing insertion of the abovementioned injection needle 10 and the luer-lock portion 12, and is provided with an engagement claw portion 6b that is formed to project to the inner side in the radial direction, with the edge portion thereof that is positioned on the outer circumference extending to the rear end side along the axis O. Also, a spring support portion 6c is formed further to the inside than the engagement claw portion 6b in the radial direction, extending along the axis O parallel with the engagement claw portion 6b. The surface that faces the rear end side further to the inside than the spring support portion 6c in the radial direction serves as a spring receiving surface 6d. The coil spring 20 as a biasing member is mounted in a compressed state between this spring receiving surface 6d and the spring abutting portion 15 that is formed on the cylindrical tip 8.

Next, the method of assembling and method of using the combined container-syringe 1 shall be described.

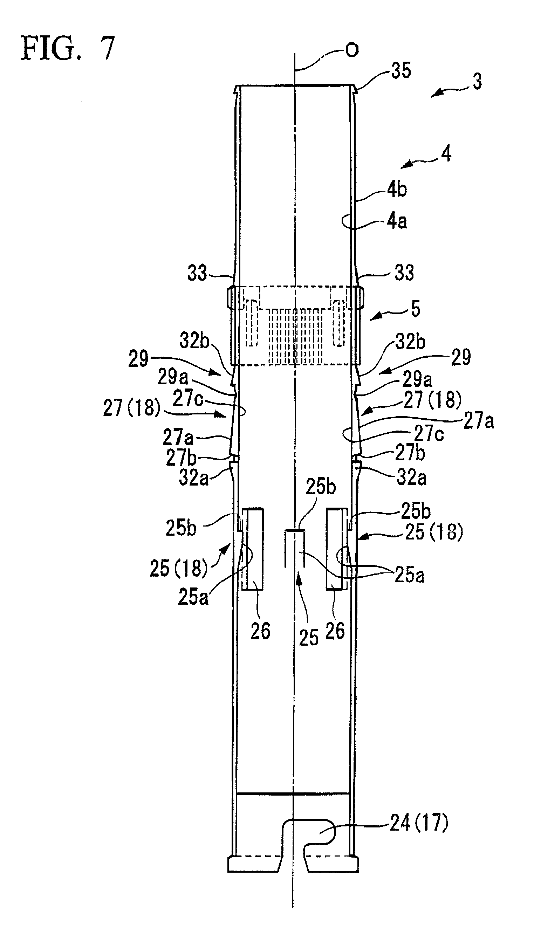

First, the pressing ring 5 is fitted and slid on the outer circumferential wall 4b of the safety device body 4 from the front end side toward the rear end. The pressing ring 5 passes over the ring primary stop projections 33 due to its flexibility and the rear end of the pressing ring 5 makes contact and stops on the sloped surface of the ring secondary stop projection 32b. In this state, the rear end side of the pressing ring 5, as shown in FIG. 7, abuts the sloping surface of the ring secondary stop projection 32b, and the front end side abuts the ring primary stop projections 33. Thereby, it is supported by the safety device body 4 in a manner of not being moved with a slight force, and thus the safety device 3 is constituted.

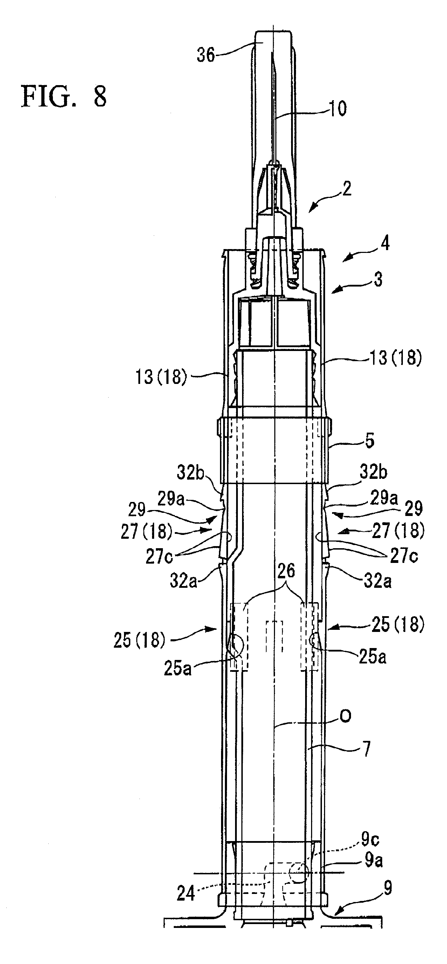

The safety device 3 is, as shown in FIG. 8, mounted on the combined container-syringe body 2 in the state of a protector 36 being mounted on the injection needle 10 with the drug solution having been filled. Specifically, when the rear end of the safety device 3 is slid from the front end side of the protector 36 of the combined container-syringe body 2 toward the rear end of the combined container-syringe body 2, the sloping surface 25a of the slip-out prevention stopper 25 abuts the engagement step portion 13a of the engagement portion 13. At this time, the sloping surface 25a flexes due to the engagement portion 13, and passes over the engagement portion 13 while expanding the inner diameter of the safety device 3. Note that since the flexibility is increased by the slits 26 being provided in the periphery of the slip-out prevention stopper 25, the slip-out prevention stopper 25 can readily pass over the engagement portion 13.

In this state, since there are no other projections on the inner circumferential wall 4a of the safety device body 4, the rear end of the safety device 3 fits on the fitting portion 9a of the finger grip 9, and moreover the safety device 3 is readily slid until the engagement projection 9c that is provided in the fitting portion 9a reaches the front end of the linear portion of the L-shaped slit 24 of the safety device 3 that extends from the rear end to the front end side. Then, by rotating either of the safety device 3 or the combined container-syringe body 2 in the circumferential direction, the engagement projection 9c and the L-shaped slit 24 engage, and the safety device 3 becomes engaged on the outer circumference of the cylinder 7 of the combined container-syringe body 2, that is, the safety device 3 becomes engaged in a first position that is a position separated from the injection needle 10.

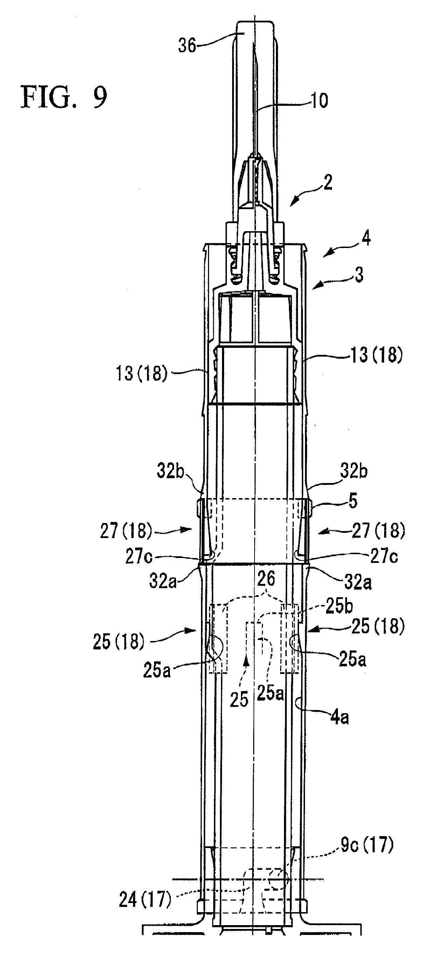

Next, when a force is applied so as to push the pressing ring 5 in the direction of the rear end, the pressing ring 5 passes over the sloping surface of the ring secondary stop projection 32b to move in the rear end direction on the outer circumference of the safety device body 4, and moves further in the rear end direction while abutting the outer circumferential surface 27a of the return prevention stopper 27. The movement of the pressing ring 5 is stopped by contact with the ring secondary stop projection 32a. At this time, the outer circumferential surface 27a of the return prevention stopper 27 is pressed inward in the radial direction by the inner circumferential wall of the pressing ring 5, and the return prevention stopper 27 flexes in the manner of a flat spring toward the inner side of the safety device body 4, with the recessed groove 29a of the connection location 29 serving as a fulcrum point. Thereby, as shown in FIG. 9, the inner circumferential surface 27c of the return prevention stopper 27 slopes so as to cause the inner circumferential wall 4a to decrease in diameter toward the rear end direction, and projects from the inner circumferential wall 4a of the safety device body 4.

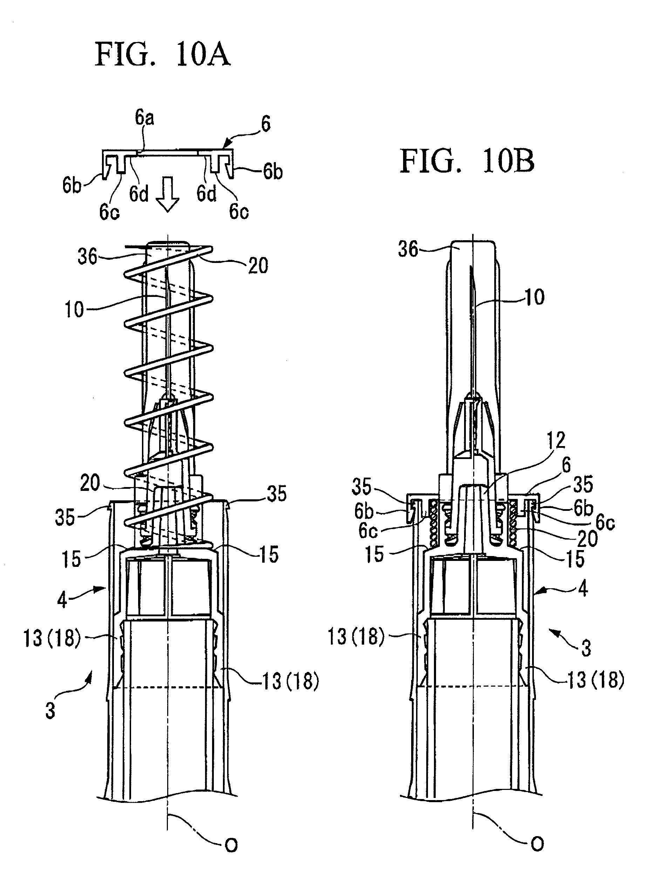

In this state, as shown in FIG. 10A, the coil spring 20 is inserted along the axis O so as to cover the injection needle 10 and the luer-lock portion 12 and the rear end of the coil spring 20 abuts the spring abutting portion 15 of the cylindrical tip 8. Then, the spring receiving member 6 is pushed from the front end of the coil spring 20 to the rear end side so as to cause the coil spring 20 to contract while the front end of the coil spring 20 abuts the spring receiving surface 6d and the outer circumferential portion of the front end of the coil spring is supported from the side by the spring supporting portion 6c. Then, the coil spring 20 contracts to its limit and the engagement claw portion 6b of the spring receiving member 6 engages with the engagement edge portion 35 that is formed on the front end of the safety device body 4, whereby the spring receiving member 6 and the safety device 3 are integrated. This state is shown in FIG. 10B.

Note that at this time, the safety device 3 is biased in the front end direction of the combined container-syringe body 2 by the coil spring 20 that is mounted in the compressed state between the spring abutting portion 15 of the cylindrical tip 8 and the spring receiving member 6. However, in this state, since the safety device 3 is engaged at the abovementioned first position by the abovementioned first retaining member 17, the safety device 3 does not move inadvertently.

The drug solution is administered to the patient by the combined container-syringe 1 constituted as above. In the state of not mounting the protector 36 on the combined container-syringe 1 after use, the combined container-syringe body 2 or the safety device 3 is rotated in the predetermined direction to release the engagement of the safety device 3 at the first position by the first retaining member 17. Then, engagement of the L-shaped slit 24 of the safety device 3 and the engagement projection 9c of the finger grip 9 is released, and the safety device 3 moves toward the front end of the combined container-syringe body 2 by the biasing force of the coil spring 20. At this time, the inner circumferential surface 27c of the return prevention stopper 27 abuts the engagement end portion 13b of the engagement portion 13, but since the safety device 3 itself is constituted with a flexible material, the inner circumferential surface 27c of the return prevention stopper 27 passes over the engagement portion 13 by flexing the inner circumferential surface 27c by the engagement portion 13 to expand the inner circumference of the safety device 3.

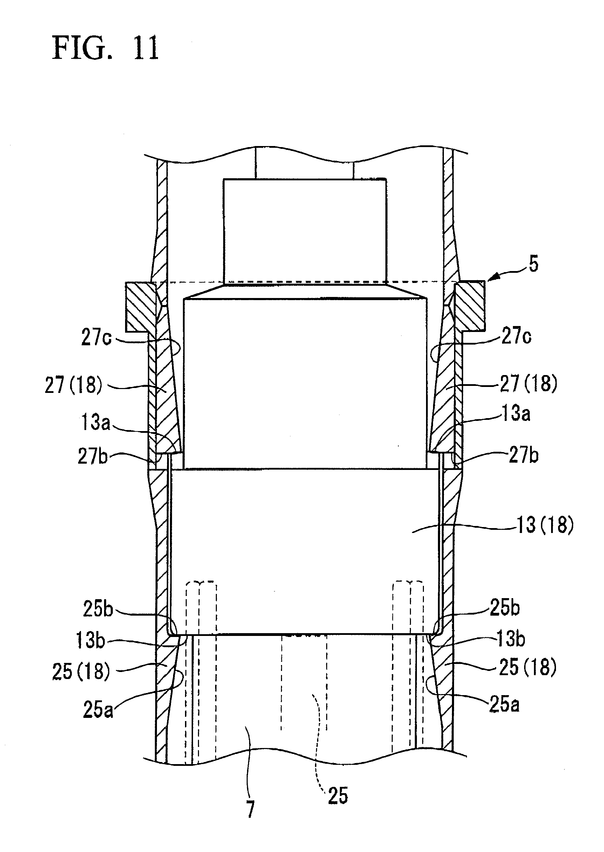

Afterward, as shown in FIG. 1, when the safety device 3 moves to a second position that is a position at which the periphery of the injection needle 10 is completely covered with the safety device 3, the engagement surface 25b of the slip-out prevention stopper 25 and the engagement end portion 13b of the engagement portion 13 engage, and further movement of the safety device 3 toward the front end is prevented, as shown in detail in FIG. 11. Moreover, in this state, even when attempting to move the safety device 3 in the rear end direction, since the engagement surface 27b of the return prevention stopper 27 and the engagement step portion 13a of the engagement portion 13 engage, such movement is prevented. That is, once the injection needle 10 is covered with the safety device 3, since the engagement portion 13 is fixed on front end side and rear end side by the slip-out prevention stopper 25 and the return prevention stopper 27, the safety device 3 is engaged at the second position by the second retaining member 18 that is constituted by the engagement portion 13, the slip-out prevention stopper 25, and the return prevention stopper 27. As a result, the safety device 3 does not separate from the combined container-syringe body 2 as a result of proceeding too far to the front end side, and neither does it move in the direction of the rear end and cause the injection needle 10 to be exposed.

As described above, according to the combined container-syringe 1 of the embodiment, when the engagement of the safety device 3 at the first position that is the position at which the safety device 3 is separated from the injection needle 10 by the first retaining member 17 is released, the coil spring 20 expands in the direction of the front end of the combined container-syringe body 2 with respect to the spring abutting portion 15 of the cylindrical tip 8. Accompanying this, the coil spring 20 causes the spring receiving member 6 and the safety device 3 to move in the direction of the front end of the combined container-syringe body 2, and the safety device 3 is fixed by the second retaining member 18 at the second position at which the safety device 3 covers the injection needle 10. Accordingly, it is possible to reliably cause the safety device 3 to move to a position that covers the injection needle 10, and it is possible to ensure safety by reliably covering the injection needle 10 with the safety device 3 after injection.

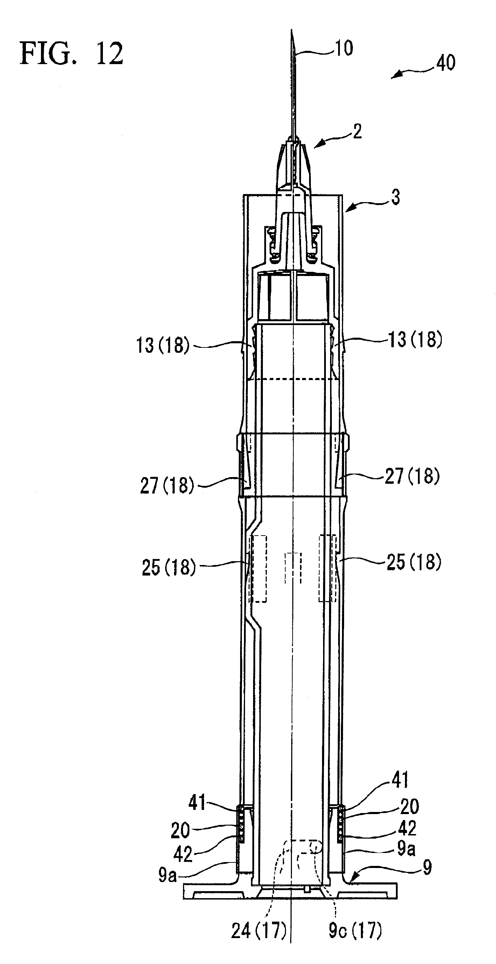

The combined container-syringe 1 of the embodiment of the present invention was described above in detail, but some design modifications are possible without being limited to them as long as not departing from the technical ideas of the present invention. For example, as a modification example, as shown in FIG. 12, the coil spring 20 may be mounted in a compressed state between the safety device 3 and the finger grip 9.

In a combined container-syringe 40 of this modification example, a step portion facing the rear end side that is formed on the inner wall surface of the safety device 3 by expanding the rear end of the safety device 3 in diameter serves as a spring receiving portion 41, and a step portion formed by reducing the front end side of the fitting portion 9a of the finger grip 9 in diameter serves as a spring abutting portion 42. When mounting the safety device 3 on the combined container-syringe body 2, the coil spring 20 is mounted in a compressed state so as to be sandwiched between the spring receiving portion 41 of the safety device 3 and the spring abutting portion 42 of the finger grip 9. The safety device 3 is engaged on the outer periphery of the cylinder 7 by the first retaining member 17, that is, the safety device 3 is engaged at a first position that is a position at which the safety device 3 is separated from the syringe 10, whereby the safety device 3 is biased toward the front end direction with respect to the combined container-syringe body 2.

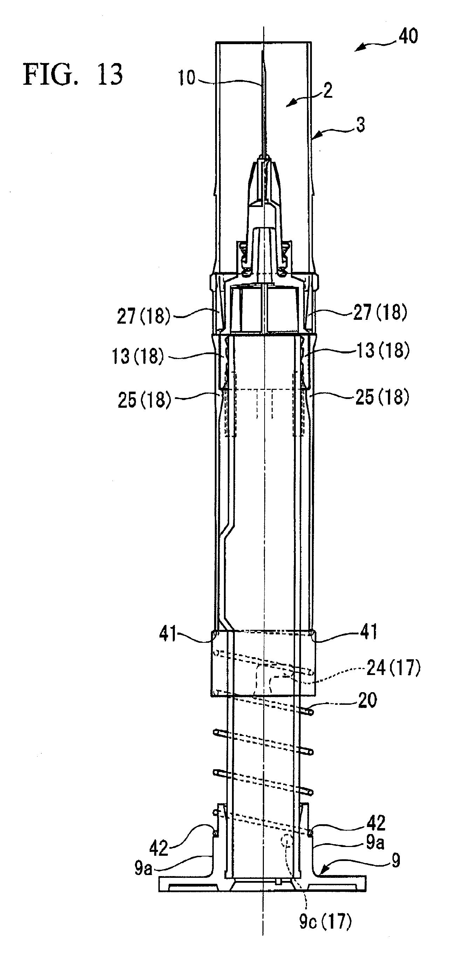

After use of the combined container-syringe 1, when the engagement of the safety device 3 at the abovementioned first position by the first retaining member 17 is released, the coil spring 20 expands toward the front end of the combined container-syringe body 2 with respect to the spring abutting portion 42 of the finger grip 9. Accompanying this, the coil spring 20 causes the safety device 3 to move in the direction of the front end of the combined container-syringe body 2, and so the safety device 3 is moved to a second position that is the position at which it covers the injection needle 10 as shown in FIG. 13. Moreover, the safety device 3 is fixed by the second retaining member 18 at this second position. Thereby, it is possible to ensure safety by reliably covering the injection needle 10 with the safety device after injection.

Note that the combined container-syringe 1 described in FIG. 1 to FIG. 11 and the combined container-syringe 40 of the modification example described in FIG. 12 and FIG. 13 used in the explanation show a dual chamber combined container-syringe having a bypass path 7a in the middle of the cylindrical cylinder 7, but the safety device 3 as mentioned above is not limited to a dual chamber combined container-syringe, and it of course may also be applied to a single chamber combined container-syringe that does not have the bypass path 7a.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A combined container-syringe comprising:
a cylindrical cylinder (7) that is filled with a drug solution;
a cylindrical tip (8) provided at the front end side of the cylinder;
an injection needle (10) mounted on the front end of the cylindrical tip;
a finger grip (9) that is provided on the rear end side of the cylinder;
a cylindrical-shaped safety device (3) that is mounted in a slidable manner on the outer periphery of the cylinder, and covers the injection needle when it has moved in the front end direction;
a retaining member (17, 18) that selectively engages the safety device at a first position that is away from the injection needle and a second position that covers the injection needle; and
a biasing member (20) that biases the safety device in the direction of the second position.

2. The combined container-syringe according to claim 1, wherein
the biasing member is a coil spring; and
in the state of the safety device being fixed to the outer periphery of the cylinder, the coil spring is mounted in a compressed state between the cylindrical tip and a spring receiving member that is provided at the front end of the safety device.

3. The combined container-syringe according to claim 1, wherein
the biasing member is a coil spring; and
in the state of the safety device being fixed to the outer periphery of the cylinder, the coil spring is mounted in a compressed state between the finger grip and a spring receiving portion that is formed on an inner circumferential wall surface on the rear end side of the safety device.

4. The combined container-syringe according to any one of claims 1 through 3, wherein the retaining member comprises:
a first retaining member that engages the safety device in a disengagable manner at the first position; and
a second retaining member that engages the safety device at the second position.
